(19) Europäisches Patentamt
European Patent Office
Office européen des brevets

(11) Veröffentlichungsnummer: **0 222 167**
**A2**

(12) **EUROPÄISCHE PATENTANMELDUNG**

(21) Anmeldenummer: **86114003.6**

(22) Anmeldetag: **09.10.86**

(51) Int. Cl.4: **B01J 31/18** , **C07D 301/12** , **C07D 303/04**

(30) Priorität: **06.11.85 DE 3539268**

(43) Veröffentlichungstag der Anmeldung: **20.05.87 Patentblatt 87/21**

(84) Benannte Vertragsstaaten: **AT BE CH DE FR GB IT LI NL SE**

(71) Anmelder: **Degussa Aktiengesellschaft**
**Weissfrauenstrasse 9**
**D-6000 Frankfurt am Main 1(DE)**

(72) Erfinder: **Schmidt, Manfred, Dr.**
**Unter-Haitzergasse 19**
**D-6460 Gelnhausen(DE)**
Erfinder: **Prescher, Günter, Dr.**
**Liesingstrasse 2**
**D-6450 Hanau 9(DE)**
Erfinder: **Müller, Helmut**
**Ziegelstrasse 8**
**D-6463 Freigericht 1(DE)**

(54) **Verfahren zur katalytischen Epoxidation von Olefinen mit Wasserstoffperoxid.**

(57) Die Erfindung betrifft ein Verfahren zur katalytischen Epoxidation von Olefinen mit Wasserstoffperoxid in Gegenwart eines Molybdänkomplexes bzw. Molybdän-oxo-Komplexes mit Oktaethylporphyrin oder mit gegebenenfalls substituierten Liganden der Formel 5, 10, 15, 20-Tetraphenylporphyrin oder 5, 10, 15, 20-Tetra(4-pyridyl)-porphyrin, wobei der Komplex jeweils am Zentralatom ein Anion trägt, oder in Gegenwart von µ-Oxo-[Mo-oxo-(5,10,15,20-tetraphenylporphyrin)]$_2$, von trans-Diperoxomolybdän(VI)porphyrin oder eines cis-Dioxoporphyrinatomolybdän(VI)-Komplexes in homogener Phase oder in einem Zweiphasensystem.

EP 0 222 167 A2

# Verfahren zur katalytischen Epoxidation von Olefinen mit Wasserstoffperoxid

Die Erfindung betrifft ein Verfahren zur katalytischen Epoxidation von Olefinen mit Wasserstoffperoxid in Anwesenheit eines Übergangsmetallporphyrinkomplexes, der gegebenenfalls in 5, 10, 15, 20-Stellung des Porphyrinringes arylsubstituiert ist und bei dem ein erforderlicher Ladungsausgleich durch ein Anion erfolgt.

Olefinoxide (Oxirane) sind Verbindungen von beträchtlicher industrieller Bedeutung. Sie finden Verwendung auf dem Gebiet der Lacke, zur Herstellung von Polyethern, Polyurethanen, Epoxidharzen, Detergentien, Glykolen und einer Vielzahl von organischen Zwischenprodukten (siehe US-PS 2 412 136 sowie DE-AS 11 39 477).

Zur Epoxidation von Olefinen sind schon verschiedene Verfahren bekannt. So lassen sich Oxirane nach der Chlorhydrinmethode durch Umsetzung von Olefinen mit Chlor oder Natriumhypochlorit im alkalischen Medium und nachfolgender Behandlung mit Basen herstellen. Ein grundsätzlicher Nachteil dieses Verfahrens besteht in der Bildung salzhaltiger umweltbelastender Abwässer und unerwünschter chlorierter Nebenprodukte (siehe Ullmann's Enzyklopädie der technischen Chemie, Band 10, Seite 565 (3. Auflage).

Ein weiterer Prozeß beruht auf der Umsetzung von Olefinen mit organischen Hydroperoxiden in Gegenwart eines Katalysators (siehe DE-AS 14 68 012). Dieses zweite Syntheseprinzip hat den entscheidenden Nachteil, daß aufgrund der Stöchiometrie der Epoxidationsreaktion das üblicherweise teure organische Hydroperoxid (ROOH, wobei R z. B. einen niedermolekularen Rest, wie t-Butyl oder Cumyl, bedeuten kann), während der Reaktion gemäß

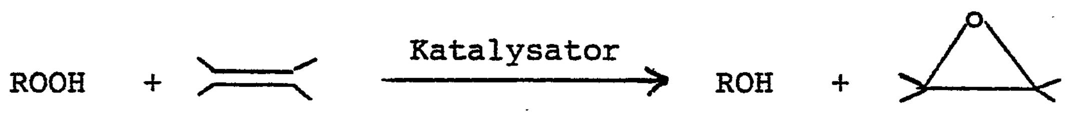


in große Mengen des entsprechenden Alkohols (ROH) umgewandelt wird. Falls der entsprechende Alkohol nicht verwertet werden kann, muß er vom Verfahrensprodukt abgetrennt und entsorgt oder über mehrere Verfahrensstufen in das entsprechende Hydroperoxid zurückverwandelt werden, wodurch das Epoxidationsverfahren auch in wirtschaftlicher Hinsicht aufwendig wird.

Ein weiteres Verfahren beruht auf der Verwendung von organischen Persäuren, die man durch Luftoxidation der entsprechenden Aldehyde oder aus Carbonsäuren mit Wasserstoffperoxid erhält (siehe BE-PS 535 068). Der Einsatz dieser organischen Percarbonsäuren ist wegen deren Zersetzlichkeit stets mit einem Risiko behaftet und erfordert deshalb aufwendige Vorsichtsmaßnahmen in bezug auf Verfahrensführung und Apparateaufbau. Zusätzlich entstehen bei Epoxidierungen mit organischen Persäuren immer große Mengen der entsprechenden Carbonsäuren, die in stöchiometrischer Menge oder überstöchiometrischer Menge abgetrennt und entsorgt oder zurückgeführt werden müssen.

Die geschilderten Nachteile lassen sich durch Verwendung von Wasserstoffperoxid als Epoxidationsmittel beheben, da hierbei nach der Theorie neben dem Epoxidationsprodukt nur Wasser anfallen sollte. Da die Reaktivität des Wasserstoffperoxids gegenüber Olefinen schwach ist, werden Epoxidationen mit diesem Reagens unter Verwendung von Katalysatoren durchgeführt. Nur für wenige Olefine sind Katalysatoren wie Molybdän-und Wolframverbindungen geeignet. In diesem Zusammenhang wird beispielsweise hingewiesen auf GB-PS 837 464, bei welchem die in J.A.C.S., Band 59, Seiten 2342 bis 2344 (1937) beschriebenen verschiedenen Metallkatalysatoren verwendet werden, auf US-PS 2 786 854, wonach Wolframsäure eingesetzt wird, auf US-PS 2 833 787, wonach saure Salze von Metallen aus der Gruppe VI des Periodensystems der Elemente, z.B. von Wolfram oder Molybdän, angewandt werden, auf BE-PS 860 776, wonach Wolfram-und Molybdänhaltige Verbindungen verwendet werden, auf US-PS 3 993 673, wonach Arsen-haltige Katalysatoren verwendet werden, auf US-PS 3 953 362, wonach ein Molybdän-haltiger Katalysator angewandt wird, auf US-PS 4 026 908, wonach Quecksilberderivate plus eine Molybdän-, Wolfram-, Vanadin-oder Titan-Verbindung angewandt wird, auf US-PS 3 806 467, wonach organische und anorganische Zinnverbindungen plus organische oder anorganische Verbindungen, die Molybdän, Wolfram, Vanadin, Selen oder Bor enthalten, eingesetzt werden, auf Bull. Chem. Soc. Jap. 42, Seiten 1604 (1969), wonach Selendioxid angewandt wird und auf US-PS 3 778 451, wonach Molybdän-, Wolfram-, Vanadin-, Niob-, Tantal-, Uran-und Rheniumverbindungen eingesetzt werden.

Diese Stoffe sind zwar katalytisch aktiv, doch haben aus verschiedenen Gründen die damit betriebenen Verfahren keinen Eingang in die Technik gefunden. In Verbindung mit Wasserstoffperoxidlösungen wird durch sie entweder das Wasserstoffperoxid rasch zersetzt oder die Eopoxidations geschwindigkeit unwirtschaftlich verlängert. Verfahren mit diesen Katalysatoren sind auch insoweit problematisch, als neben dem gewünschten Epoxidationsprodukt häufig größere Mengen an Nebenprodukten, wie Diole und Ketone gebildet werden, deren Abtrennung erhebliche Schwierigkeiten bereiten kann.

Es sind auch schon Versuche unternommen worden, Verfahren zur katalytischen Epoxidation von Olefinen mit anderen Epoxidationsmitteln unter Verwendung von Metallporphyrinkomplexen als Katalysatoren durchzuführen. Als Epoxidationsmittel wurden hierbei Verbindungen wie Jodosobenzol PhJO (Groves, J.T.; Nemo, T.E.; Myers, R.S, J. Am. Chem. Soc., 101, 1032 (1979), Alkalimetallhypochlorit, wie NaOCl oder LiOCl (Guilmet, E.; Meunier, B.; Tetrahedron Lett. 1980, 4449) sowie organische Hydroperoxide, wie t-Butylhydroperoxid oder Cumolhydroperoxid (Ledon, H.J.; Durbut, P.; Varescon, F., J. Am. Chem. Soc. 103, 3601 (1981) eingesetzt. Als zur Umsetzung mit diesen Epoxidationsmitteln geeignete Metallkatalysatoren sind z. B. das Chloro-Eisen (III)-tetraphenylporphyrin (ClFe(III)(TPP)), das Chloro-Mangan (III)-tetraphenylporphyrin (ClMn(III)(TPP)) oder das Chloro-Chrom(III)-tetraphenylporphyrin (ClCr(III)(TPP)) vorgeschlagen worden. Mangan(III)-tetraphenylporphyrin wurde auch bereits mit Wasserstoffperoxid als Oxidationsmittel eingesetzt (Renaud, J.-P.; Battioni, P.; Bartoli,J.F.; Mansuy, D., J. Chem. Soc., Chem. Commun., 1985, 888). Allerdings wirken diese stark zersetzend auf $H_2O_2$, so daß die bezüglich Wasserstoffperoxid erreichbaren Selektivitäten nur sehr gering sind.

Auch Oxo-Metallporphyrinkomplexe, wie Oxo-chloro(5,10,15, 20-tetraphenylporphyrin)-Molybdän(V) -(O=MO(V)(TPP)Cl) sind in Verbindung mit organischen Hydroperoxiden schon vorgeschlagen worden. Ein Versuch, anstelle eines organischen Hydroperoxids Wasserstoffperoxid mit einem Katalysator der Zusammensetzung Oxo(5,10,15,20-tetraphenylporphyrin)-Molybdän(V) -methoxid zur Epoxidierung des Olefins Cyclohexen zu verwenden, schlug jedoch fehl: Es konnte keine Epoxidation beobachtet werden - (F.Varescon, These, L'Université Claude Bernard-Lyon I, 1982).

Demgegenüber wurde gefunden, daß die katalytische Epoxidation von Olefinen mit Wasserstoffperoxid mit hoher Selektivität gelingt, wenn das Olefin in Gegenwart eines Molybdän-komplexes bzw. Molybdän-oxo-Komplexes mit Oktaethylporphyrin oder mit Liganden der Formel

-5, 10, 15, 20-Tetraphenylporphyrin,

-5, 10, 15, 20-Tetra(4-pyridyl)-porphyrin,

bei denen gegebenenfalls jeweils Wasserstoffatome an den Phenyl-bzw. Pyridylgruppen ein-oder mehrmals substituiert sind durch Halogen, Hydroxy, Carboxy, Cyano, Rhodano, Nitro, $C_1$-$C_6$-Alkyl, Trihalogenmethyl, $C_1$-$C_6$-Alkoxjy, $C_1$-$C_6$-Alkansulfonyloxy, Aminocarbonyl, einen oder zwei $C_1$-$C_6$-Alkylreste enthaltendes Aminocarbonyl, $C_1$-$C_6$-Alkylcarbonyl, Amino, Di-$C_1$-$C_6$-Alkylamino, ($C_1$-$C_6$-Alkyl)$_3$N, $C_1$-$C_6$-Alkanoylamino, $C_1$-$C_6$-Alkyl-$C_1$-$C_6$-alkanoylamino, $C_1$-$C_6$-Alkansulfonylamino, $C_1$-$C_6$-Alkyl-$C_1$-$C_6$-alkansulfonylamino, Aminosulfonyl, einen oder zwei $C_1$-$C_6$-Alkylreste enthaltendes Aminosulfonyl, $C_1$-$C_6$-Alkoxysulfonyl (-$SO_2$-O-$C_1$-$C_6$-Alkyl), Sulfo oder $C_1$-$C_6$-Alkansulfonyl und zwei dieser Reste auch die Methylendioxygruppe sein können, wobei der Komplex jeweils am Zentralatom ein Anion aus der Reihe $F^-$, $Cl^-$, $Br^-$, $J^-$, $CH_3O^-$, $C_2H_5O^-$, $C_2H_7O^-$, t-$C_4H_9O^-$, $HO^-$, $AcO^-$, $SCN^-$, $C_6H_5O^-$, pyridyl$^-$ trägt

oder in Gegenwart

-einer dimeren Dioxoverbindung der Formel $\mu$-Oxo[Mo-oxo-(5,10,15,20-tetraphenylporphyrin)]$_2$,

-einer Bisperoxoverbindung der Formel trans-Diperoxomolybdän(VI)porphyrin oder

-eines cis-Dioxoporphyrinatomolybdän(VI)-Komplexes

in homogener Phase oder in einem Zweiphasensystem mit Wasserstoffperoxid umsetzt.

Durch sterische und elektronische Effekte der Substituenten am Phenyl-bzw. Pyridylrest des 5, 10, 15, 20-Tetraphenylporphyrins bzw. 5, 10, 15, 20-Tetra(4-pyridyl)porphyrins kann man, angepaßt an das jeweilige Olefin, die katalytischen Eigenschaften steuern.

Nach dem erfindungsgemäßen Verfahren können Olefine entsprechend der allgemeinen Formel

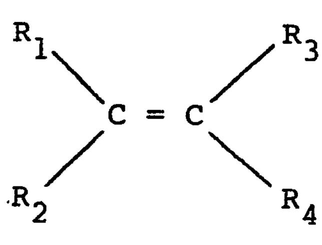

epoxidiert werden, wobei $R_1$ bis $R_4$ identisch oder verschieden sein können und sowohl Wasserstoff oder

einen linearen oder verzweigten Alkylrest mit 1 bis 30 Kohlenstoffatomen oder einem Cycloalkylrest mit 3 bis 12 Kohlenstoffatomen oder Aromaten bzw. Heteroaromaten mit 3 bis 30 C-Atomen, die als Heteroatome z. B. ein-oder mehrere O-, N-oder S-Atome enthalten können, bedeuten. $R_1$ und $R_2$ oder $R_3$ und $R_4$ können auch durch funktionelle Gruppen substituiert sein, welche im Reaktionsmilieu stabil sind, wie z. B. durch Hydroxy-, Chloro-, Fluoro-, Bromo-, Jodo-, Nitro-; Methoxy-, Alkoxy-, Amino-, Carbonyl-, Ester-, Amido-, Nitrilo-Gruppen. Sie können auch ungesättigt sein, d. h. daß Polyolefine, wie z. B. Diene, Triene und andere Verbindungen mit Doppelbindungen ebenfalls in der vorliegenden Erfindung verwendet werden können, seien sie konjugiert oder nicht.

Unter dieser Voraussetzung kommen unter den Olefinen, welche nach dem vorliegenden Verfahren epoxidiert werden können, beispielsweise die folgenden in Betracht:

Ethylen, Propylen, die Butene, Butadien, die Pentene, Isopren, Hexen(1), Hexen(3), Hepten(1), Okten(1), Diisobutylen, Nonen(1), Tetradecen(1), Pentamyrcen, Camphen, Undecen(1), Dodecen(1), Tridecen(1), Tetradecen(1), Pentadecen(1), Hexadecen(1), Heptadecen(1), Oktadecen(1), Nonadecen(1), Eikosen(1), die Trimeren und Tetrameren des Propylens, die Polybutadiene, die Polyisoprene, Styrol, $\alpha$ -Methylstyrol, Divinylbenzl, Inden, Stilben, Cyclopenten, Cyclohexen, Cyclohepten, Cycloocten, Cyclooctadien, Cyclododecen, Cyclododekatrien, Dicyclopentadien, Methylencyclopropan, Methylencyclopentan, Methylencyclohexan, Vinylcyclohexen, Methallylketon, Allylchlorid, Allylbromid, Arylsäure, Methacrylsäure, Crotonsäure, Vinylessigsäure, Crotylchlorid, Methallylchlorid, die Dichlorbutene, Allylalkohol, Allylkarbonat, Allylacetat, die Alkyle der Acrylate und Methacrylate, Diallylmaleat, Diallylphthalat, die ungesättigten Öle wie Sojaöl, die ungesättigten Fettsäuren wie Ölsäuren, Linolensäure, Balidinsäure, Erucasäure, Oleostearinsäure, Myristinsäure, Palmitinölsäure, Ricinolsäure und deren Ester.

Ein Vorteil der Erfindung besteht darin, daß sich hierbei das als Reaktant benötigte Wasserstoffperoxid in allen handelsüblichen Formen verwenden läßt, nämlich in Form wäßriger Wasserstoffperoxidlösungen mit einem Wasserstoffperoxidgehalt von 30 bis 90 Gew.% oder als reines Wasserstoffperoxid, stärker verdünntes Wasserstoffperoxid, in organischen Lösungsmitteln gelöstes wasserfreies Wasserstoffperoxid oder in Form von Verbindungen, die unter den Reaktionsbedingungen Wasserstoffperoxid freisetzen - (Metallperoxide, wie Magnesium-oder Zinkperoxid sowie Wasserstoffperoxid-Anlagerungsverbindungen (Peroxohydrate), z. B. von Natriumcarbonat, Natriumpyrophosphat und Harnstoff).

Besonders vorteilhaft ist, wenn man als Reaktionsmedium ein organisches Lösungsmittel oder ein Lösungsmittelgemisch verwendet, welches einen Übertritt von als wäßrige Lösung eingesetztem Wasserstoffperoxid in die organische Phase gestattet.

Als organische Lösungsmittel können dafür z. B. Alkyl-oder Cycloalkylester von gesättigten aliphatischen Carbonsäuren mit einer Kohlenstoffzahl von 4 -8 (siehe z.B. DE-PS 32 25 307, Seite 5), Methylenchlorid, Dioxan, tert.-Butanol, Tetrahydrofuran, Benzol, Äthanol, Chloroform und Methanol verwendet werden.

Als Lösungsmittelgemische kommen z. B. Kombinationen aus einem oder mehreren der obengenannten Carbonsäureester mit Wasser, Methylenchlorid, Dioxan, tert.-Butanol, Tetrahydrofuran, Benzol, Äthanol, Chloroform und/oder Methanol in Frage.

Für die Lösung von wasserfreiem Wasserstoffperoxid hat sich ein Einsatz von Alkyl-oder Cycloalkylestern von gesättigten aliphatischen Carbonsäuren mit einer Kohlenstoffzahl von 4-8 besonders bewährt.

Die anzuwendenden Mengen an Katalysator, die im erfindungsgemäßen Verfahren eingesetzt werden, können in einem weiten Bereich liegen. Die im Einzelfall anzuwendende Katalysatorkonzentration kann, entsprechend dem Typ der gewählten, gemäß Erfindung vorgesehenen Molybdänporphyrinverbindung sowie entsprechend der Reaktivität des jeweils umzusetzenden Olefins gewählt werden. Sie liegt in einem Konzentrationsbereich, der im allgemeinen 1/2000 bis 1/2 mol, vorzugsweise 1/1000 bis 1/5 mol pro mol Wasserstoffperoxid beträgt.

Nach einer anderen vorteilhaften Ausführungsform der Erfindung können die ohnehin hohen Selektivitätsraten durch Zusatz von geringen Mengen, vorzugsweise von 0,1 bis 10 mol eines heterocyclischen Amins, bezogen auf 1 mol des Kata lysators, insbesondere von äquimolaren Mengen, einer Verbindung aus der Familie des Pyridins, wie 2,6-Dimethylpyridin, 2,6-Ditertiärbutylpyridin, 3,5-Dimethylpyridin sowie die drei Picoline, die 4-Halogenopyridine sowie die Salze des 2,2-Bipyridyls, oder des Imidazols, z. B. das Imidazol selbst, verbessert werden. Die Konzentration des Olefins im Reaktionssystem ist nicht kritisch, das molare Verhältnis zwischen Olefin und Wasserstoffperoxid kann 1 : 30 bis 30 : 1 betragen.

Die Reaktionstemperaturen können in einem breiten Bereich liegen. Sie hängen ab von der jeweiligen Aktivität des verwendeten Katalysators, der Reaktivität des verwendeten Olefins, der Neigung des gewünschten Oxirans zur Ringöffnung und der Art des Lösungsmittels. Sie liegen im allgemeinen bei 0 bis 150, vorzugsweise 20 bis 120, insbesondere 40 bis 80° C. Die Reaktionszeiten sind kurz und liegen normalerweise bei 1 bis 24 Stunden. Die Reaktionen können unter Atmosphärendruck oder bei höheren Drucken durchgeführt werden, solange das Reaktionssystem in flüssiger Phase gehalten werden kann.

Vorzugsweise wird in einem Druckbereich zwischen 1 und 50 bar gearbeitet.

Die erfindungsgemäß zur Durchführung des Verfahrens vorgesehenen Katalysatoren sind nach den bekannten Literaturmethoden in großer Reinheit zugänglich (H. Ledon et al. Inorg. Chim. Acta, 31 (1978) L 393; E.B. Fleischer et al., J.Am.Chem.Soc. 92, 5518 (1970); Y.Murakami et al., Chem. Lett. 1977, 1281; J.W. Buchler et al., Chem. Ber., 1973, 106, 2710; Liebigs Ann. Chem., 1971, 745, 135; Inorg. Nucl. Chem. Lett., 1972, 8, 1073, H. Stoppa, Dissertation, RTWH Aachen, 1976; R. Weiss et al., Inorg. Chem. Acta, 1976, 19, L 570; F. Varescon, These, L'Université Claude Bernard-Lyon, 1982).

Die verschiedenen Porphyrinliganden werden, sofern sie nicht käuflich sind, nach Adler et al., J.Org.Chem. 32, 476 (1967) bzw. Adler et al. J.Heterocycl .Chem. 5, 669 (1968) dargestellt und sofern erforderlich, von Chlorin (Porphyrin mit 1 teilhydrierten Pyrrolglied) befreit (K.M. Smith et al. Tetrahedron Lett. 30, 2887, (1973).

Die mit der Erfindung erzielbaren Vorteile sind:

-Hohe Selektivität (kaum Nebenprodukt)

-Niedrige Katalysatorkonzentration

-Hohe chemische Stabilität des Katalysators, insbesondere gegenüber dem Epoxidationsmittel

-Keinerlei $H_2O_2$-Zersetzung

-Aus dem Epoxidationsmittel entsteht nur Wasser

-Leichte Abtrennbarkeit und Wiederverwendbarkeit des Katalysators

- Einfacher Verfahrensgang

Die Erfindung wird im folgenden anhand von Ausführungsbeispielen näher erläutert.

Vorbemerkung zu den einzelnen Beispielen:

Die in den Ausführungsbeispielen verwendeten, nach der Literatur hergestellten Katalysatoren werden zur Epoxidation unterschiedlicher olefinischer Ausgangsstoffe mit Wasserstoffperoxid gemäß der Erfindung wie folgt eingesetzt:

Man stellt eine Lösung aus Olefin, Katalysator und Lösungsmittel her, erwärmt sie auf eine Temperatur im Bereich von 20 -100° C und versetzt mit Wasserstoffperoxid (30 bis 90 Gew.%) -Version I -oder man stellt eine Lösung aus Olefin, Wasserstoffperoxid (30 bis 90 Gew.%) und Lösungsmittel her und versetzt diese dann mit Katalysator; dann wird unter Rühren und Erwärmen umgesetzt -Version II.

In einer abgewandelten Ausführungsform, die sich auf die Versionen I und II gleichermaßen anwenden läßt, wird den vorgelegten Komponenten vor Zugabe der restlichen Komponente noch eine kleine Menge eines heterocyclischen Amins, z. B. aus der Familie des Pyridins oder Imidazols zugesetzt. Bei den eingesetzten Olefinen kann unter atmosphärischem Druck gearbeitet werden. Im Verlaufe der Umsetzung werden Proben entnommen und auf ihren Gehalt an Epoxid bzw. $H_2O_2$ analysiert. Die Menge an gebildeten Epoxiden wird entweder durch Gaschromatographie oder Titration, diejenige an Wasserstoffperoxid durch übliche Titration mit Cer(IV)-sulfat ermittelt. Die bei den Versuchen erhaltenen Ergebnisse gehen aus der folgenden Tabelle hervor, wobei die Selektivität wie folgt definiert ist:

$$\text{Selektivität (\%)} = \frac{\text{Mol gebildetes Epoxid}}{\text{Mol umgesetztes } H_2O_2} \times 100$$

| Beispiel Nr. | Olefin (mmol) | $H_2O_2$ (mmol) | Katalysator | (mmol) | Reaktionsbeding. Solvent (ml) | Temp. (°C) | Zeit (h) | Umsatz $H_2O_2$ (%) | Selektiv. (%) |
|---|---|---|---|---|---|---|---|---|---|
| 1 | Cyclohexen (72) | 31,3% in nPAC[1)] (24) | o = Mo(TPP) | $24 \times 10^{-3}$ | nPAC / Py[2)] 30 / 0.5 | 60 | 7 | 6,9 | 93,3 |
| 2 | Cyclohexen (72) | 85 % in $H_2O$ (24) | o = Mo(TPP) | $24 \times 10^{-3}$ | nPAC / Py/ $H_2O$ 30/0.5/0.15 | 60 | 24 | 26 | 99,8 |
| 3[3)] | Cyclohexen (72) | – | o = Mo(TPP) | $24 \times 10^{-3}$ | nPAC / Py 30 / 0.5 | 60 | 6 | 0 | 0 |
| 4 | 2-Methyl-2-buten (72) | 31,3 % in nPAC (24) | μ-o $[o{=}Mo(TPP)]_2$ | $24 \times 10^{-2}$ | nPAC/γ-Pic[4)] 30 / 0.1 | 60 | 24 | 92,7 | 57,4 |
| 5 | Allylalkohol (72) | 31,3 % in nPAC (24) | μ-o $[o{=}Mo(TPP)]_2$ | $24 \times 10^{-2}$ | nPAC/γ-Pic 30 / 0.1 | 60 | 24 | 56,1 | 10,7 |
| 6 | 1,5-COD[5)] (72) | 31,3 % in nPAC (24) | μ-o $[o{=}Mo(TPP)]_2$ | $24 \times 10^{-2}$ | nPAC/γ-Pic 30 / 0.1 | 60 | 24 | 81,2 | 50,6 |
| 7 | Cyclohexen (72) | 31,3 % in nPAC (24) | μ-o $[o{=}Mo(TPP)]_2$ | $24 \times 10^{-2}$ | nPAC 30 | 60 | 6 | 95,6 | 10,1 |
| 8 a | 1,5-COD (72) | 31,3 % in nPAC (24) | o = Mo(TPP)Cl | $24 \times 10^{-2}$ | nPAC 30 | 60 | 24 | 95,8 | 44,3 |
| 8 b | 1,5-COD (22,5) | 31,3 % in nPAC (7,5) | o = Mo(TPP)Cl* | $7{,}5 \times 10^{-2}$ | nPAC 10 | 60 | 24 | 90,3 | 44,0 |

1) Essigsäurepropylester
2) Pyridin
3) Kontrollbeispiel
4) γ-Picolin
5) 1,5-Cyclooctadien
* Wiederverwendung des Katalysators nach Beispiel 8 a

| Beispiel Nr. | Olefin (mmol) | $H_2O_2$ (mmol) | Katalysator | (mmol) | Reaktionsbeding. Solvent (ml) | Temp. (°C) | Zeit (h) | Umsatz $H_2O_2$ (%) | Selektivität (%) |
|---|---|---|---|---|---|---|---|---|---|
| 9[3] | 1,5-COD (72) | 31,3 % in nPAC (24) | - | - | nPAC 30 | 60 | 24 | 0 | 0 |
| 10 | 1,5-COD (72) | 31,3 % in nPAC (24) | μ-o[o=Mo(TPP)]$_2$ | $24 \times 10^{-2}$ | nPAC/$CH_2Cl_2$ 2.0 / 30 | 40 | 24 | 71,3 | 22,2 |
| 11 | 1,5-COD (72) | 31,3 % in nPAC (24) | o = Mo(TPP)$OCH_3$ | $24 \times 10^{-2}$ | nPAC 30 | 60 | 6 | 90,9 | 10,4 |
| 12 | 1,5-COD (72) | 31,3 % in nPAC (24) | Bisperoxo Mo(VI) (TPP) | $24 \times 10^{-2}$ | nPAC/γ-Pic 30 / 0.1 | 60 | 24 | 69,0 | 45,4 |
| 13 | 1,5-COD (36) | 31,3 % in nPAC (12) | o = Mo(TPP)SCN | $12 \times 10^{-2}$ | n PAC/γ-Pic 15 / 0.05 | 60 | 24 | 88,8 | 50,5 |
| 14 | 1,5-COD (72) | 85 % in $H_2O$ (24)[2] | μ-o[o=Mo(TPP)]$_2$ | $24 \times 10^{-2}$ | nPAC/$H_2O$ 30 / 0.15 | 60 | 24 | 83,8 | 51,0 |
| 15 | 1,5-COD (72) | 31,3 % in nPAC (24) | cis-Dioxo-Mo (VI)(TPP) | $24 \times 10^{-3}$ | n PAC 30 | 60 | 24 | 75,4 | 24,7 |
| 16 | 1,5-COD (18) | 31,3 % in nPAC (6) | o=Mo(OEP)[6]$OCH_3$ | $6,4 \times 10^{-2}$ | n PAC 10 | 60 | 6 | 97,6 | 39,1 |
| 17 | 1,5-COD (72) | 31,3 % in nPAC (24) | o=Mo(T-4pyP)[7] $OCH_3$ | $24 \times 10^{-2}$ | n PAC 30 | 60 | 6 | 95,5 | 42,3 |

6) Oktaethylporphyrin

7) 5,10,15,20-Tetra(4-pyridyl)-porphyrin

| Beisp. | Olefin (mmol) | $H_2O_2$ (mmol) | Katalysator | (mmol) | Reaktionsbeding. Solvent (ml) | Temp. (°C) | Zeit (h) | Umsatz $H_2O_2$ (%) | Selektiv. (%) |
|---|---|---|---|---|---|---|---|---|---|
| 18 | 1,5-COD (22,5) | 31,3 % in BPE[12] (7,5) | o = Mo(TpTP)[8]Cl | $7,5 \times 10^{-2}$ | BPE 10 | 60 | 2 | 98,6 | 16,6 |
| 19 | 1,5-COD (72) | 31,3 % in PME[13] (24) | o = Mo(ToClPP)[9]Cl | $24 \times 10^{-2}$ | PME 30 | 60 | 2 | 98,9 | 42,6 |
| 20 | 1,5-COD (72) | 31,3 % in nPAC (24) | BisperoxoMo(VI) (TPP) | $24 \times 10^{-2}$ | nPAC/Dioxan/γ-Pic 2.0/30/0.1 | 60 | 5 | 28,6 | 57,8 |
| 21 | 1,5-COD (72) | 85 % in $H_2O$ (24) | BisperoxoMo(VI) (TPP) | $24 \times 10^{-2}$ | Dioxan/$H_2O$/γ-Pic 30/0.15/0.1 | 60 | 5 | 10,0 | 86,3 |
| 22 | 1,5-COD (36) | 31,3 % in nPAC (12) | BisperoxoMo(VI) (TPP) | $12 \times 10^{-2}$ | nPAC/t-BuOH[10] 1.0/15/0.05 | 60 | 24 | 30,7 | 58,4 |
| 23 | 1,5-COD (36) | 31,3 % in nPAC (12) | BisperoxoMo(VI) (TPP) | $12 \times 10^{-2}$ | nPAC/THF[11]/γ-Pic 1.0/15/0.05 | 60 | 24 | 32,0 | 59,0 |
| 24 | 1,5-COD (36) | 31,3 % in nPAC (12) | BisperoxoMo(VI) (TPP) | $12 \times 10^{-2}$ | nPAC/Benzol/γ-Pic 1.0/15/0.05 | 60 | 5 | 89,4 | 9,6 |

8) 5,10,15,20-Tetra(p-tolyl)-porphyrin
9) 5,10,15,20-Tetra(o-chlorphenyl)-porphyrin
10) tert.-Butanol
11) Tetrahydrofuran
12) Buttersäurepropylester
13) Pivalinsäuremethylester

## Ansprüche

1. Verfahren zur katalytischen Epoxidation von Olefinen mit Wasserstoffperoxid in Anwesenheit eines Übergangs-metallporphyrinkomplexes, der gegebenenfalls in 5, 10, 15, 20-Stellung des Porphyrinringes arylsubstituiert ist und bei dem ein erforderlicher Ladungsausgleich durch ein Anion erfolgt, dadurch gekennzeichnet, daß man das Olefin in Gegenwart eines Molybdänkomplexes bzw. Molybdän-oxo-Komplexes mit Oktaethylporphyrin oder mit Liganden der Formel

-5, 10, 15, 20-Tetraphenylporphyrin,

-5, 10, 15, 20-Tetra(4-pyridyl)-porphyrin

bei denen gegebenenfalls jeweils Wasserstoffatome an den Phenyl-bzw. Pyridylgruppen ein-oder mehrmals substituiert sind durch Halogen, Hydroxy, Carboxy, Cyano, Rhodano, Nitro, $C_1$-$C_6$-Alkyl, Trihalogenmethyl, $C_1$-$C_6$-Alkoxy, $C_1$-$C_6$-Alkansulfonyloxy, Aminocarbonyl, einen oder zwei $C_1$-$C_6$-Alkylreste enthaltendes Aminocarbonyl, $C_1$-$C_6$-Alkylcarbonyl, Amino, Di-$C_1$-$C_6$-Alkylamino, ($C_1$-$C_6$-Alkyl)$_3$N, $C_1$-$C_6$-Alkanoylamino, $C_1$-$C_6$-Alkyl-$C_1$-$C_6$-alkanoylamino, $C_1$-$C_6$-Alkansulfonylamino, $C_1$-$C_6$-Alkyl-$C_1$-$C_6$-alkansulfonylamino, Aminosulfonyl, einen oder zwei $C_1$-$C_6$-Alkylreste enthaltendes Aminosulfonyl, $C_1$-$C_6$-Alkoxysulfonyl(-$SO_2$-O-$C_1$-$C_6$-Alkyl), Sulfo oder $C_1$-$C_6$-Alkansulfonyl und zwei dieser Reste auch die Methylendioxygruppe sein können, wobei der Komplex jeweils am Zentralatom ein Anion aus der Reihe $F^-$,$Cl^-$, $Br^-$, $J^-$, $CH_3O^-$, $C_2H_5O^-$, $CH_3H_7O^-$, t-$C_4H_9O^-$, $HO^-$, $AcO^-$, $SCN^-$, $C_6H_5O^-$, pyridyl trägt,

oder in Gegenwart

-einer dimeren Dioxoverbindung der Formel $\mu$-Oxo[Mo-oxo-(5,10,15,20-tetraphenylporphyrin)]$_2$,

-einer Bisperoxoverbindung der Formel trans-Diperoxomolybdän(VI)porphyrin oder

-eines cis-Dioxoporphyrinatomolybdän(VI)-Komplexes

in homogener Phase oder in einem Zweiphasensystem mit Wasserstoffperoxid umsetzt.

2. Verfahren nach Anspruch 1, dadurch gekennzeichnet, daß man als Reaktionsmedium ein organisches Lösungsmittel oder ein Lösungsmittelgemisch verwendet, welches einen Übertritt von als wäßrige Lösung eingesetztem Wasserstoffperoxid in die organische Phase gestattet.

3. Verfahren nach Anspruch 1 oder 2, dadurch gekennzeichnet, daß man bei Einsatz von wasserfreiem Wasserstoffperoxid als organische Lösungsmittel Alkyl-oder Cycloalkylester von gesättigten aliphatischen Carbonsäuren mit einer Kohlenstoffzahl von 4 -8 einsetzt.

4. Verfahren nach den Ansprüchen 1 -3, dadurch gekennzeichnet , daß die Umsetzung in Gegenwart von 0,1 bis 10 mol, vorzugsweise 0,5 -5 mol, bezogen auf 1 mol des Katalysators, einer Verbindung aus der Familie des Pyridins oder Imidazols erfolgt.

5. Verfahren nach Anspruch 4, dadurch gekennzeichnet, daß $\gamma$-Picolin oder Imidazol eingesetzt wird.